# EUROPÄISCHE PATENTANMELDUNG

(11) **EP 4 837 607 A1**
(43) Veröffentlichungstag der Anmeldung: **11.11.2026**
(21) Anmeldenummer: 26176695.0
(22) Anmeldetag: 05.05.2026
(51) Int. Cl.: A61M 16/12, A61M 16/00

(54) **VORRICHTUNG FÜR DIE INHALATIVE STICKSTOFFMONOXID-THERAPIE**

(30) Priorität: 06.05.2025 DE 102025117351
(71) Anmelder: EKU Elektronik GmbH, 56291 Leiningen (DE)
(72) Erfinder: Köbrich, Rainer, 35321 Laubach (DE); Stemann, Daniel, 33469 Simmern (DE)
(74) Vertreter: Münzel, Joachim R.

(57) **Zusammenfassung**

Eine Vorrichtung für die inhalative Stickstoffmonoxid-Therapie umfasst ein Dosiergerät (2), das mit einer Steuereinrichtung (14) und einer Einrichtung (12) zum Messen des Gasmengenstroms ausgerüstet ist, sowie ein mit einer Druckgasflasche (8) verbindbares Druckreduzierventil (6), an dem ein anströmseitiger Primärdruck erfasst werden kann, der der Steuereinrichtung übermittelt wird. Die Steuereinrichtung (14) ist so eingerichtet, dass sie aus den gemessenen Werten für den Primärdruck und für den Gasmengenstrom des Therapiegases eine Information über das Füllvolumen einer angeschlossenen Druckgasflasche berechnet.

## Beschreibung

Die Erfindung betrifft eine Vorrichtung für die inhalative Stickstoffmonoxid-Therapie, mit einem Dosiergerät, das mit einer Dosierleitung zum Einleiten eines Stickstoffmonoxid enthaltenden Therapiegases in das Atemgas eines Patienten, einer in der Dosierleitung integrierten Messeinrichtung zum Messen zum Messen des durch die Dosierleitung geführten Gasmengenstroms, einem in der Dosierleitung integrierten Steuerventil zum Einstellen des Gasmengenstroms, sowie einer mit der Messeinrichtung und dem Steuerventil datenverbundenen Steuereinrichtung zum Regeln des Gasdurchflusses ausgerüstet ist, und mit einem an eine Druckgasflasche anschließbaren Druckreduzierventil, das mit Mitteln zum Erfassen eines druckgasflaschenseitigen Primärdrucks ausgerüstet und sekundärdruckseitig mit der Dosierleitung strömungsverbunden ist.

Bei der inhalativen Stickstoffmonoxid-Therapie (iNO-Therapie) wird Stickstoffmonoxid über die Atemwege verabreicht. Dabei wird ein Stickstoffmonoxid (NO) enthaltendes Therapiegas in den Beatmungsstrom eingespeist, der dem Patienten über eine Atemgaszuleitung zugeführt wird. Hierzu kommt in der Regel ein Dosiergerät zum Einsatz, an dem die jeweils vorgesehene Konzentration an Therapiegas im Atemgas durch eine Regelung des Mengenstroms des eingespeisten Therapiegases voreingestellt wird. Als Quelle für das Therapiegas kommt in der Regel eine Druckgasflasche zum Einsatz, die mittels eines Druckreduzierventils an das Dosiergerät angeschlossen wird. Reicht der Inhalt einer Druckgasflasche nicht aus, um den Patienten für die volle Dauer der Therapie mit Therapiegas zu versorgen, muss diese noch während der laufenden Therapie durch eine volle Druckgasflasche ersetzt werden. Es sind auch bereits Dosiergeräte am Markt, die für einen dualen Betrieb mit zwei wechselweise zur Therapiegasversorgung eingesetzten Druckgasflaschen ausgelegt sind und die somit eine kontinuierliche Versorgung des Patienten mit Therapiegas während einer Therapie sicherstellen können.

Dosiergeräte für die iNO-Therapie verfügen heute häufig über Möglichkeiten, die verbleibende Zeitdauer, für die noch genügend Therapiegas zur Durchführung der eingestellten Therapie in der jeweils angeschlossenen Druckgasflasche zur Verfügung steht, zu bestimmen und auf einer Anzeigeeinheit des Dosiergeräts anzuzeigen. Dazu wird der Mengenstrom des entnommenen Therapiegases sowie der Primärdruck am Druckreduzierventil erfasst und hieraus ein Prognosewert für die verbleibende Zeit bis zur Entleerung der Druckgasflasche berechnet. Die entsprechenden Rechenroutinen gehen dabei jedoch in der Regel von Druckgasflaschen mit einem Füllvolumen von 10 l bis 11 l aus, was einem heute häufig verwendeten Standard entspricht. Weicht das tatsächliche Füllvolumen der angeschlossenen Druckgasflasche hiervon ab, wird eine fehlerhafte Restdauer angezeigt, mit der Folge, dass es entweder zu Ausfällen in der Therapiegasversorgung des Patienten (bei einer zu lang angezeigten Restdauer) oder zu einer Verschwendung von noch in der Flasche enthaltenem Therapiegas (bei einer zu kurz angezeigten Restdauer) kommen kann.

Aufgabe der vorliegenden Erfindung ist es somit, eine Vorrichtung für die inhalative Stickstoffmonoxid-Therapie zu schaffen, bei dem die für eine laufende Therapie verbleibende Zeitdauer bis zur Entleerung einer angeschlossenen Druckgasflasche zuverlässig und unabhängig von der jeweiligen Flaschengröße angegeben werden kann.

Gelöst ist diese Aufgabe durch eine Vorrichtung mit den Merkmalen des Patentanspruchs 1. Vorteilhafte Ausgestaltungen der Erfindung sind in den Unteransprüchen angegeben.

Erfindungsgemäß ist eine Vorrichtung der eingangs genannten Art und Zweckbestimmung also dadurch gekennzeichnet, dass die Steuereinrichtung mit den Mitteln zum Erfassen des Primärdrucks am Druckreduzierventil datenverbunden ist und die Steuereinrichtung so eingerichtet ist, dass während einer laufenden Therapie die Werte für die zeitliche Änderung des Primärdrucks und für den durch die Dosierleitung strömende Gasmengenstrom an Therapiegas erfasst und hieraus eine Information über das Füllvolumen der angeschlossenen Druckgasflasche gewonnen wird.

Im Betrieb der erfindungsgemäßen Vorrichtung wird ein Stickstoffmonoxid enthaltendes Therapiegas aus der angeschlossenen Druckgasflasche in einem an der Dosiereinrichtung voreingestellten Mengenstrom entnommen. Mit der Entnahme sinkt allmählich der Primärdruck am Druckreduzierventil, der im Wesentlichen dem Druck in der angeschlossenen Druckgasflasche entspricht. Mengenstrom und Primärdruck werden mit geeigneten Einrichtungen erfasst und die erfassten Werte an die Steuereinrichtung des Dosiergeräts übermittelt. In der Steuereinrichtung ist bevorzugt ein Rechenprogramm eingespeichert, dessen Algorithmus aus der entnommenen Menge an Therapiegas und der mit der Entnahme einhergehenden Änderung des Primärdrucks eine Information über das Füllvolumen der angeschlossenen Druckgasflasche gewinnt. Die Information über das Füllvolumen kann einem marktüblichen Flaschenvolumen (beispielsweise 2l, 5l, 10l, 11l oder 20l) zugeordnet und entsprechend auf einer Anzeigeeinheit visualisiert werden, beispielsweise auf einer Anzeigeeinheit des Dosiergeräts selbst oder auf einer externen Anzeige, etwa auf einem Mobilgerät.

Bevorzugt ist die Steuereinrichtung zudem so eingerichtet, dass sie aus den gemessenen Werten eine Prognose für eine Restzeitdauer berechnet, über die der am Druckreduzierventil eingestellte Sekundärdruck noch aufrecht erhalten werden kann, ohne die Druckgasflasche wechseln zu müssen. Bei Dosiergeräten, die nur an eine einzelne Druckgasflasche angeschlossen werden können, entspricht dies der Zeitdauer, die noch für eine unterbrechungsfreie Therapie zur Verfügung steht. Auch die Restzeitdauer kann an einer Anzeigeeinrichtung des Dosiergeräts und/oder einer externen Anzeigeeinrichtung visualisiert werden.

Weiterhin ist die Steuereinrichtung zweckmäßigerweise so eingerichtet, dass bei Unterschreiten eines vorgegeben Grenzwerts für den Primärdruck ein akustisches oder optisches Warnsignal abgegeben wird, das einem Therapeuten anzeigt, dass für eine Fortsetzung der Therapie ein Wechsel der Druckgasflasche erforderlich ist.

Eine abermals vorteilhafte Ausgestaltung der Erfindung sieht vor, dass das Dosiergerät für einen dualen Betrieb mit zwei Druckgasflaschen als Therapiegasquellen ausgelegt ist. Dazu ist die Dosierleitung mit zwei jeweils an eine Druckgasflasche anschließbaren, jeweils mit Mitteln zum Erfassen eines druckgasflaschenseitigen Primärdrucks ausgerüsteten Druckreduzierventilen strömungsverbunden. Über eine Wechselschaltung in der Dosierleitung, beispielsweise einem elektronisch ansteuerbaren Zweiwegeventil, können die Druckgasflaschen nacheinander zum Einsatz gebracht werden. Die Steuereinrichtung des Dosiergeräts ist bei dieser Ausgestaltung mit den Mitteln zum Erfassen des Primärdrucks an beiden Druckreduzierventilen datenverbunden und so eingerichtet, dass die Werte des jeweiligen Primärdrucks erfasst werden und hieraus sowie aus dem Mengenstrom des entnommenen Therapiegases eine Information über das Füllvolumen der jeweils angeschlossenen Druckgasflasche gewonnen und ggf. an einer Anzeigeeinrichtung visualisiert wird*.*

Die erfindungsgemäße Vorrichtung ist zur Abgabe eines Stickstoffmonoxidenthaltenden Therapiegases bestimmt und geeignet. Beim Therapiegas handelt es sich beispielsweise um reines Stickstoffmonoxid oder um ein Gasgemisch, das neben Stickstoffmonoxid wenigstens ein weiteres Gas enthält, insbesondere Stickstoff und/oder ein Edelgas; beispielsweise besteht das Therapiegas aus einem Volumenanteil zwischen 450 ppm und 1000 ppm Stickstoffmonoxid, Rest Stickstoff.

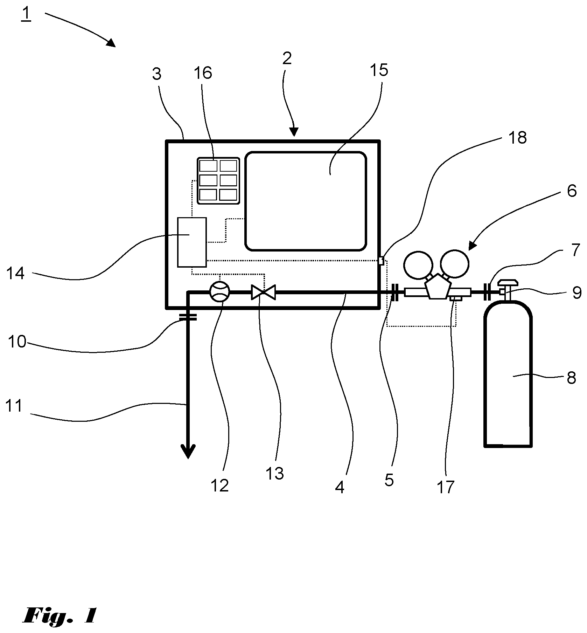

Anhand der Zeichnung soll ein Ausführungsbeispiel der Erfindung näher erläutert werden. Die einzige Zeichnung (Fig. 1) zeigt schematisch eine erfindungsgemäße Vorrichtung für die inhalative Stickstoffmonoxid-Therapie.

Die in Fig. 1 gezeigte Vorrichtung 1 umfasst ein Dosiergerät 2, durch dessen Gehäuse 3 eine Dosierleitung 4 geführt ist. Die Dosierleitung 4 ist außerhalb des Gehäuses 3 an einem Anschlussstutzen 5 mit einem Druckreduzierventil 6 strömungsverbunden, das über einen Eingangsanschluss 7 verfügt, an dem eine Druckgasflasche 8 mit ihrem Flaschenventil 9 angeschlossen werden kann. Die Druckgasflasche 8 dient zur Bereitstellung eines Stickstoffmonoxid enthaltenden Therapiegases und weist ein marktübliches Füllvolumen von beispielsweise 2 Liter, 5 Liter, 10 Liter, 11 Liter oder 20 Liter auf. Über einen weiteren Anschlussstutzen 10 ist die Dosierleitung 4 mit einer Therapiegaszuleitung 11 strömungsverbunden, die in an sich bekannter und hier nicht gezeigter Weise in eine Atemgaszuleitung zum Zuführen eines Atemgases an einen Patienten einmündet.

In der Dosierleitung 4 ist ein Messgerät 12 zum Messen eines durch die Dosierleitung 4 geführten Gasmengenstroms sowie ein Steuerventil 13 zum Einstellen dieses Gasmengenstroms integriert. Messgerät 12 und Steuerventil 13 stehen mit einer elektronischen Steuereinrichtung 14 in Datenverbindung. Das Dosiergerät 2 verfügt des Weiteren über eine Anzeigeeinheit 15 und ein Bedienpanel 16, die beide ebenfalls mit der Steuereinrichtung 14 in Datenverbindung stehen und die im Übrigen auch beispielsweise in einem Touchscreen zusammengefasst sein können. Am Bedienpanel 16 können verschiedene, für die Therapie wichtige Parameter, wie beispielsweise Mengenstrom des Therapiegases, Art des Therapiegases oder Dauer der Therapie eingegeben werden; an der Anzeigeeinheit 15 können die entsprechenden Werte, ebenso wie weitere Daten, beispielsweise Daten eines Patienten, visualisiert werden.

Das Druckreduzierventil 6 verfügt über eine Messeinrichtung 17 zum Erfassen eines anströmseitigen Drucks (Primärdruck) am Druckreduzierventil 6. Die Messeinrichtung 17 ist über ein Datenkabel oder kabellos mit einer Schnittstelle 18 des Dosiergeräts 2 verbunden, bei der es sich beispielsweise um eine RS 232-Schnittstelle, einen USB-Anschluss oder um eine kabellose Schnittstelle, beispielsweise um einen Bluetooth-Empfänger handelt. Die Schnittstelle 18 ist ebenfalls mit der Steuereinrichtung 14 datenverbunden.

Im Betrieb der Vorrichtung 1 wird vor Beginn einer Therapie das Flaschenventil 9 geöffnet und am Bedienpanel 16 eine Therapiegaskonzentration eingestellt. Dadurch strömt Therapiegas aus der Druckgasflasche 8 durch die Dosierleitung 4 und die Therapiegasleitung 11 zum Patienten (hier nicht gezeigt). Am Druckreduzierventil 6 liegt anströmseitig ein Primärdruck an, der dem Druck in der Druckgasflasche 8 entspricht und durch die Gasentnahme allmählich abnimmt. Der abströmseitige Druck (Sekundärdruck) am Druckreduzierventil 6 ist niedriger als der Primärdruck und wird für die Dauer der Therapie in der Regel auf einem konstanten Wert gehalten. Der Primärdruck wird von der Messeinrichtung 17 kontinuierlich oder in vorgegebenen Zeitabständen gemessen und die gemessenen Werte der Steuereinrichtung 14 übermittelt. Der durch die Dosierleitung 4 geführte Gasmengenstrom wird am Messgerät 12 erfasst und die Werte gleichfalls der Steuereinrichtung 14 übermittelt. Mittels des Steuerventils 13 kann der Gasmengenstrom ggf. geregelt werden.

Die Steuereinrichtung 14 ist so eingerichtet, dass sie aus den gemessenen Werten für den Gasmengenstrom und dem Primärdruck eine Information über das Füllvolumen der angeschlossenen Druckgasflasche 8 ermittelt. Beispielsweise ist in der Steuereinrichtung 14 ein Programm eingespeichert, dessen Algorithmus den im Verlauf der Therapie abfallenden Primärdruck am Druckreduzierventil 6 mit der gleichzeitig verabreichten Therapiegasmenge korreliert und hieraus eine Information über das Flaschenvolumen der angeschlossenen Druckgasflasche 8 errechnet. Der Wert für das so bestimmte Flaschenvolumen kann an der Anzeigeeinheit 15 angezeigt werden. Der Wert kann auch mit eingespeicherten Werten für bekannte Flaschenvolumina (beispielsweise 2l, 5l, 10l, 11l, 20l) verglichen und ein passendes Flaschenvolumen an der Anzeigeeinheit 15 angezeigt werden.

Das in der Steuereinrichtung 14 eingespeicherte Programm kann darüber hinaus vorsehen, dass aus den ermittelten Werten für Druck, Flaschenvolumen und Gasmengenstrom eine Restzeitdauer ermittelt wird, die den Zeitraum angibt, über den die Therapie bei gleichbleibendem Gasmengenstrom fortgesetzt werden kann. Die aktuelle Restzeitdauer kann an der Anzeigeeinheit 15 visualisiert werden. Weiterhin kann die Steuereinrichtung 14 so eingerichtet sein, dass bei Unterschreiten eines vorgegebenen Mindestwertes für den Primärdruck ein Warnsignal abgegeben wird, das ebenfalls an der Anzeigeeinheit 15 angezeigt werden kann und einem Therapeuten vermittelt, dass die Druckgasflasche 8 vollständig oder weitgehend entleert ist und gegen eine volle ausgetauscht werden muss.

In einer alternativen, hier nicht gezeigten Ausgestaltung der Erfindung ist eine Dosiereinheit für einen dualen Betrieb mit zwei Druckgasflaschen ausgelegt. Die Dosiereinheit weist eine Dosierleitung auf, in der eine Wechselschaltung angeordnet ist, beispielsweise ein elektronisch ansteuerbares Zweiwegeventil, an der zwei Druckreduzierventile zum Anschließen jeweils einer Druckgasflasche an die Dosierleitung anschließbar sind. Beide Druckreduzierventile sind mit einer Einrichtung zum Erfassen des jeweiligen Primärdrucks ausgerüstet, und beide dieser Erfassungseinrichtungen stehen über eine Schnittstelle mit einer Steuereinrichtung der Dosiereinheit in Datenverbindung. Die Steuereinrichtung ist so eingerichtet, dass für beide Druckgasflaschen in der oben beschriebenen Weise eine Information über das jeweilige Füllvolumen ermittelt werden kann.

### Bezugszeichenliste

- 1: Vorrichtung
- 2: Dosiergerät
- 3: Gehäuse
- 4: Dosierleitung
- 5: Anschlussstutzen
- 6: Druckreduzierventil
- 7: Eingangsanschluss
- 8: Druckgasflasche
- 9: Flaschenventil
- 10: Anschlussstutzen
- 11: Therapiegaszuleitung
- 12: Messgerät
- 13: Steuerventil
- 14: Steuereinrichtung
- 15: Anzeigeeinheit
- 16: Bedienpanel
- 17: Messeinrichtung
- 18: Schnittstelle

## Patentansprüche

1. Vorrichtung für die inhalative Stickstoffmonoxid-Therapie,
mit einem Dosiergerät (2), das mit einer Dosierleitung (4) zum Einleiten eines Stickstoffmonoxid enthaltenden Therapiegases in das Atemgas eines Patienten, mit einer in der Dosierleitung (4) integrierten Messeinrichtung (12) zum Messen des durch die Dosierleitung (4) geführten Gasmengenstroms, mit einem in der Dosierleitung (4) integrierten Steuerventil (13) zum Einstellen des Gasdurchflusses, sowie mit einer, mit der Messeinrichtung (12) und dem Steuerventil (13) datenverbundenen Steuereinrichtung (14) zum Regeln des Gasdurchflusses ausgerüstet ist, und mit einem an eine Druckgasflasche (8) anschließbaren Druckreduzierventil (6), das mit Mitteln (17) zum Erfassen eines druckgasflaschenseitigen Primärdrucks ausgerüstet und sekundärdruckseitig mit der Dosierleitung (4) strömungsverbunden ist,
**dadurch gekennzeichnet,**
**dass** die Steuereinrichtung (14) mit den Mitteln (17) zum Erfassen des Primärdrucks datenverbunden ist und so eingerichtet ist, dass während einer laufenden Therapie die Werte für die zeitliche Änderung des Primärdrucks und der durch die Dosierleitung (4) strömenden Gasmengenstrom an Therapiegas erfasst und hieraus eine Information über das Füllvolumen der angeschlossenen Druckgasflasche (8) gewonnen wird.

2. Vorrichtung nach Anspruch 1, **dadurch gekennzeichnet, dass** die Steuereinrichtung (14) so eingerichtet ist, dass aus dem ermittelten Wert des Füllvolumens und dem kumulativen Wert des Gasmengenstroms eine Information über die noch für eine Therapie zur Verfügung stehende Restzeitdauer ermittelt wird.

3. Vorrichtung nach Anspruch 1 oder 2, **dadurch gekennzeichnet, dass** die Steuereinrichtung (14) so eingerichtet ist, dass bei Unterschreiten eines vorgegeben Grenzwerts für den Primärdruck ein Warnsignal abgegeben wird.

4. Vorrichtung nach einem der vorhergehenden Ansprüche, **dadurch gekennzeichnet, dass** die Dosierleitung (4) des Dosiergeräts (2) mit zwei jeweils an eine Druckgasflasche (8) anschließbaren, jeweils mit Mitteln (17) zum Erfassen eines druckgasflaschenseitigen Primärdrucks ausgerüsteten Druckreduzierventilen (6) verbunden ist, und in der Dosierleitung (4) eine Wechselschaltung vorgesehen ist, die eine wechselweise Strömungsverbindung der Druckreduzierventile (6) mit der Dosierleitung (4) erlaubt, wobei die Steuereinrichtung (14) des Dosiergeräts mit den Mitteln (17) zum Erfassen des Primärdrucks beider Druckreduzierventile (6) datenverbunden ist und so eingerichtet ist, dass die Werte des jeweiligen Primärdrucks und des Gasmengenstroms erfasst und hieraus eine Information über das Füllvolumen der jeweils angeschlossenen Druckgasflasche (8) gewonnen wird.
